# EUROPÄISCHE PATENTSCHRIFT

(11) **EP 2 263 518 B1**
(45) Veröffentlichungstag und Bekanntmachung des Hinweises auf die Patenterteilung: **06.01.2016**
(21) Anmeldenummer: 10005961.7
(22) Anmeldetag: 09.06.2010
(51) Int. Cl.: A61B 1/06

(54) **Endoskop mit einem Fenster und Verfahren zum Herstellen eines Endoskops**
Endoscope with a window and method for manufacturing an endoscope
Endoscope doté d'une fenêtre et procédé de fabrication d'un endoscope

(30) Priorität: 17.06.2009 DE 102009025660
(43) Veröffentlichungstag der Anmeldung: 22.12.2010
(73) Patentinhaber: Karl Storz GmbH & Co. KG, 78532 Tuttlingen (DE)
(72) Erfinder: Bürk, André, 78054 Villingen-Schwenningen (DE); Teichtmann, Elmar, 75031 Eppingen (DE); Heseler, Stefan, 78647 Trossingen (DE)

(56) Entgegenhaltungen:
- EP-A1- 1 247 484
- DE-A1- 3 740 417
- US-A1- 2002 128 535

## Beschreibung

Die vorliegende Erfindung bezieht sich auf ein Endoskop mit einem Fenster am distalen Ende und ein Verfahren zum Herstellen eines Endoskops. Insbesondere bezieht sich die vorliegende Erfindung auf ein Endoskop, bei dem beispielsweise mittels eines schwenkbaren Prismas oder Spiegels ein Winkel zwischen der Blickrichtung und der axialen Richtung des Endoskops variiert werden kann.

Minimal-invasive Operationen haben zahlreiche Vorteile, beispielsweise eine geringere Traumatisierung und eine schnellere Genesung des Patienten, geringere Anforderungen an die Anästhesie, kürzere Verweildauern des Patienten im Operationsraum und anschließend in der Klinik sowie insgesamt geringere Kosten. Ein bei einem minimal-invasiven Eingriff verwendetes Endoskop muss gleichzeitig zahlreiche Anforderungen erfüllen. Insbesondere muss es eine hohe Zuverlässigkeit bzw. Verfügbarkeit aufweisen und trotz der thermischen Belastung beim Autoklavieren nach jedem Einsatz eine lange Lebensdauer aufweisen.

Endoskope mit einer variierbaren Blickrichtung sind bereits in den Druckschriften US 6,110,105 A, US 6,638,216 B1, US 2004/0236183 A1 und WO 01/22865 A1 beschrie- nach vielen Autoklavier-Zyklen noch hermetische dicht sein, um ein Eindringen von Feuchtigkeit in das Innere des Endoskops und die Schädigung der Optik durch eingedrungene Feuchtigkeit zu verhindern.

Die US 2002/0128535 A1 ist auf ein autoklavierbares Endoskop bezogen. Um eine hermetische Dichtheit zu erzielen und ein Eindringen von Feuchtigkeit in ein Objektiv zu verhindern, ist ein äußerer Umfang eines optischen Fensterbauteils mit Metall beschichtet und mit einem ersten metallischen Linsentubus verlötet. Um Reflexionen zu vermeiden ist ein Teil des Objektivlinsensystems in einem zweiten metallischen Linsentubus angeordnet, der eine Oberfläche mit niedrigen Reflexionsgrad aufweist. Das optische Fensterbauteil und der distale Rand des ersten metallischen Linsentubus sind in einer Öffnung einer Abdeckung des distalen Endes des Endoskops angeordnet.

Die DE 37 40 417 A1 ist auf eine Endoskopoptik bezogen, die heiß sterilisiert werden kann. In einem Zwischenraum zwischen einem Hüllrohr und einem im Hüllrohr liegenden Führungsrohr sind Lichtleitfasern für Beleuchtungszwecke angeordnet. Im Führungsrohr ist ein Optikrohr, welches ein optisches System in Form von Linsen aufnimmt, angeordnet. Zum Schutz des optischen Systems gegen Verunreinigung von außen ist ein Fenster vorgesehen, dessen umfänglicher Rand mittels Metalllot mit der Innenfläche des Führungsrohres verlötet ist.

Die EP-1247484 zeigt ein Endoskop mit den Merkmalen des Oberbegriffs des Anspruchs 1.

Insbesondere musste die Anmelderin feststellen, dass bei andersartigen Endoskopen mit ebenem, kreisförmigem oder elliptischem Fenster bewährte Fügetechniken zur dauerhaften mechanischen Verbindung des Fenster mit dem Gehäuse bzw. Mantel des Endoskops bei gekrümmten Fenstern nicht anwendbar sind. Aufgrund der Krümmung, der vergleichsweise großen Gesamtfläche und einer deutlich nicht-elliptischen Kontur rufen die Temperaturen und die Temperaturänderungsraten eines gewöhnlichen Autoklavier-Vorgangs mechanische Spannungen hervor, die nach kurzer Zeit zu Rissen oder Brüchen im Fenster oder in der Fügenaht führen.

Eine Aufgabe der vorliegenden Erfindung besteht darin, ein verbessertes Endoskop und ein verbessertes Verfahren zum Herstellen eines Endoskops mit einem Fenster, insbesondere einem gekrümmten Fenster, zu schaffen.

Diese Aufgabe wird durch den Gegenstand des unabhängigen Anspruchs gelöst.

Weiterbildungen sind in den abhängigen Ansprüchen angegeben.

Verschiedene Ausführungsformen der vorliegenden Erfindung beruhen auf der Idee, die Aufgaben der Fügenaht am Rand eines Fensters aufzuteilen in eine erste Dichtnaht zwischen Fenster und Mantel bzw. Gehäuse des Endoskops und eine zweite, hermetische Dichtnaht, die ein Eindringen von Feuchtigkeit in einen Raum, in dem die optischen Komponenten des Endoskops angeordnet sind, auch unter den Bedingungen des Autoklavierens zuverlässig verhindert. Anders ausgedrückt wird das Fenster an einen Innenkörper gefügt, der ein dazu - insbesondere hinsichtlich des Wärmeausdehnungskoeffizienten-besonders geeignetes Material aufweist. Das Fenster kann auf diese Weise eine Öffnung zu einem Hohlraum in dem Innenkörper in einer auch nach zahlreichen Autoklavier-Zyklen noch zuverlässig hermetisch dichten Weise verschließen. Das Material des Innenkörpers kann weitgehend frei gewählt werden, da er keine äußere Oberfläche des Endoskops bilden sondern von einem Mantel bzw. Gehäuse umschlossen sein kann. Nur das Material des Mantels bzw. Gehäuses muss die üblichen Anforderungen an ein Endoskops, insbesondere die der Biokompatibilität, erfüllen. Mit einer vergleichsweise einfachen Abdichtung zwischen dem Rand des Fensters und dem Rand einer entsprechenden Öffnung im Mantel bzw. im Gehäuse kann sichergestellt werden, dass der menschliche oder tierische Körper, an dem das Endoskop verwendet wird, nicht mit dem Material des Innenkörpers in Berührung kommt.

Ein Endoskop umfasst ein Fenster an einem distalen Ende des Endoskops, einen Innenkörper mit einem ersten Material am distalen Ende und einen Mantel mit einem zweiten Material am distalen Ende, wobei das Fenster und der Innenkörper gefügt, sind, und wobei eine Klebenaht oder eine Dichtung zwischen dem Rand des Fensters und dem Mantel vorgesehen ist.

Verschiedene Ausführungsformen der vorliegenden Erfindung beruhen auf der Idee, die Aufgaben der Fügenaht am Rand eines Fensters aufzuteilen in eine erste Dichtnaht zwischen Fenster und Mantel bzw. Gehäuse des Endoskops und eine zweite, hermetische Dichtnaht, die ein Eindringen von Feuchtigkeit in einen Raum, in dem die optischen Komponenten des Endoskops angeordnet sind, auch unter den Bedingungen des Autoklavierens zuverlässig verhindert. Anders ausgedrückt wird das Fenster an einen Innenkörper gefügt, der ein dazu - insbesondere hinsichtlich des Wärmeausdehnungskoeffizienten - besonders geeignetes Material aufweist. Das Fenster kann auf diese Weise eine Öffnung zu einem Hohlraum in dem Innenkörper in einer auch nach zahlreichen Autoklavier-Zyklen noch zuverlässig hermetisch dichten Weise verschließen. Das Material des Innenkörpers kann weitgehend frei gewählt werden, da er keine äußere Oberfläche des Endoskops bilden sondern von einem Mantel bzw. Gehäuse umschlossen sein kann. Nur das Material des Mantels bzw. Gehäuses muss die üblichen Anforderungen an ein Endoskop, insbesondere die der Biokompatibilität, erfüllen. Mit einer vergleichsweise einfachen Abdichtung zwischen dem Rand des Fensters und dem Rand einer entsprechenden Öffnung im Mantel bzw. im Gehäuse kann sichergestellt werden, dass der menschliche oder tierische Körper, an dem das Endoskop verwendet wird, nicht mit dem Material des Innenkörpers in Berührung kommt.

Ein Endoskop umfasst ein Fenster an einem distalen Ende des Endoskops, einen Innenkörper mit einem ersten Material am distalen Ende und einen Mantel mit einem zweiten Material am distalen Ende, wobei das Fenster und der Innenkörper gefügt sind.

Der Innenkörper, der auch als Innensegment bezeichnet werden kann, bildet insbesondere keine äußeren Oberflächen des Endoskops, so dass nur der Mantel bzw. das Gehäuse des Endoskops biokompatibel sein muss. Das erste Material, das der Innenkörper zumindest am distalen Ende aufweist, kann nun so gewählt werden, dass insbesondere seine Ausdehnung bei Temperaturerhöhungen, wie sie beim Fügen von Fenster und Innenkörpers oder beim Autoklavieren auftreten, an die des Fensters angepasst ist. Insbesondere kann die Differenz der thermischen Ausdehnungskoeffizienten des ersten Materials und eines Materials des Fensters (beispielsweise Saphir oder ein anderer Korund) kleiner sein als eine Differenz der thermischen Ausdehnungskoeffizienten des zweiten Materials und des Materials des Fensters.

Beispielsweise weist nicht rostender Stahl 1.4301 oder 1.4305 bei Temperaturen ab 20°C einen linearen Wärmeausdehnungskoeffizienten von ca. 16 x 10⁻⁶ K⁻¹ auf. Saphir ist aufgrund seiner großen Härte als Material für das Fenster besonders geeignet, weist aber einen linearen Wärmeausdehnungskoeffizienten von nur ca. 6 x 10⁻⁶ K⁻¹ auf. Vorteilhaft ist deshalb die Auswahl eines ersten Materials mit einem thermischen Ausdehnungskoeffizienten, dessen Differenz vom thermischen Ausdehnungskoeffizienten des Materials des Fensters statt ca. 10 x 10⁻⁶ K⁻¹ nur noch 5 x 10⁻⁶ K⁻¹ oder besser nur noch 2 x 10⁻⁶ K⁻¹ oder noch besser 1 x 10⁻⁶ K⁻¹ oder weniger beträgt. Bei Verwendung eines Fensters aus Saphir ist deshalb ein erstes Material mit einem linearem Wärmeausdehnungskoeffizienten von höchstens 8 x 10⁻⁶ K⁻¹ besonders geeignet, noch besser geeignet sind Materialien mit einem linearen Wärm eausdehnungskoeffizienten von höchstens 7 x 10⁻⁶ K⁻¹ oder mit einem Wärmeausdehnungskoeffizienten zwischen 5,5 x 10⁻⁶ K⁻¹ und 6,0 x 10⁻⁶ K⁻¹ oder 6,5 x 10⁻⁶ K⁻¹. Diese Anforderungen erfüllen beispielsweise NiFe-Legierungen mit einem Nickel-Massen-Antcil von ca. 42% oder NiCoFe-Legierungen mit einem Nickel-Massen-Anteil von ca. 29% und einem Cobalt-Massen-Anteil von ca. 17%. Derartige Werkstoffe werden auch als Einschmelzwcrkstoffe oder Ausdehnungswerkstoffe bezeichnet und beispielsweise von der Deutsche Nickel GmbH unter dem Markennamen Dilaton und mit den Bezeichnungen "29/18", "42" und "42 LC" vertrieben. Daneben sind jedoch auch andere Einschmelzlegierungen verwendbar.

Das Endoskop ist insbesondere ein Endoskop mit einer variierbaren Blickrichtung bzw. einem variierbaren Winkel zwischen der Blickrichtung und der axialen Richtung des Endoskops, zur Variation des Winkels zwischen Blickrichtung und axialer Richtung weist das Endoskop beispielsweise ein schwenkbares Prisma, einen schwenkbaren Spiegel oder eine schwenkbare Kamera nahe dem Fenster auf Insbesondere für ein derartiges Endoskop kann das Fenster in zumindest einer Richtung gekrümmt sein. Das Fenster kann eine Öffnung zu einem Hohlraum in dem Innenkörper hermetisch verschließen. Insbesondere für einen hermetischen Verschluss einer Öffnung zu einem Hohlraum in dem Innenkörper können das Fenster und der Innenkörper verlötet sein. Sowohl Hartlötverfahren als auch Weichlötverfahren kommen in Frage, insbesondere unter Verwendung eines Goldlots. Zwischen dem Rand des Fensters und dem Mantel kann eine Klebenaht und/oder eine Dichtung oder ein anderes flexibles Material vorgesehen sein. Zur weiteren Verringerung des Risikos eines Bruchs des Fensters durch thermisch hervorgerufene mechanische Spannungen kann das Fenster abgerundete Ecken aufweisen.

Der Mantel kann einen Abschlussabschnitt am distalen Ende und einen Schaftabschnitt umfassen, wobei der Abschlussabschnitt und der Schaftabschnitt gefügt sind. Der Schaftabschnitt ist insbesondere im Wesentlichen rohrförmig. Der Abschlussabschnitt umfasst insbesondere eine dem Fenster bzw. dessen Kontur entsprechende Öffnung. Der Abschlussabschnitt ist beispielsweise ausgebildet, um an einer Hinterschneidung an einem distalen Ende des Innenkörpers eingehängt und dann durch Schwenken um eine Achse nahe der Hinterschneidung in seine vorgesehene Lage gebracht zu werden. Alternativ ist der Abschlussabschnitt beispielsweise ausgebildet, um in einer gekrümmten oder geradlinigen linearen Bewegung, die mit einer Rotation verbunden sein kann und zumindest in ihrem letzten Abschnitt nicht parallel zur Achse des Endoskops verläuft, an das distale Ende des Endoskops angesetzt zu werden. Dabei kann der Abschlussabschnitt gleichzeitig an den Schaftabschnitt angesetzt werden, mit dem er anschließend gefügt wird. Durch eine der vorbeschriebenen Ausbildungen des Mantels kann der Innenkörper mit Ausnahme der Fläche des Fensters vollständig umschlossen werden, nachdem das Fenster und der Innenkörper gefügt sind.

Das Endoskop kann an zwei gegenüberliegenden Seiten des Fensters jeweils mehrere Lichtaustrittsöffnungen aufweisen, wobei die Lichtaustrittsöffnungen an einer Seite des Fensters versetzt zu den Lichtaustrittsöffnungen an der anderen Seite des Fensters angeordnet sind. Damit kann eine gleichmäßigere Ausleuchtung eines mit dem Endoskop betrachteten Raums ermöglicht werden. Eine symmetrische Anordnung der Lichtaustrittsöffnungen, gegebenenfalls mit der Realisierung einer gleichmäßigen Ausleuchtung durch anderweitige Maßnahmen, ist ebenfalls denkbar.

Ein Endoskop umfasst - unabhängig von aber durchaus kombinierbar mit den vorstehend beschriebenen Merkmalen des Fensters und eines Innenkörpers des Endoskops - einen Mantel mit einem Abschlussabschnitt am distalen Ende und einem Schaftabschnitt, wobei der Abschlussabschnitt und der Schaftabschnitt gefügt sind. Der Schaftabschnitt ist insbesondere im Wesentlichen rohrförmig. Der Abschlussabschnitt umfasst insbesondere eine dem Fenster bzw. dessen Kontur entsprechende Öffnung. Der Abschlussabschnitt ist beispielsweise ausgebildet, um an einer Hinterschneidung an einem distalen Ende des Innenkörpers eingehängt und dann durch Schwenken um eine Achse nahe der Hinterschneidung in seine vorgesehene Lage gebracht zu werden. Alternativ ist der Abschlussabschnitt beispielsweise ausgebildet, um in einer gekrümmten oder geradlinigen linearen Bewegung, die mit einer Rotation verbunden sein kann und zumindest in ihrem letzten Abschnitt nicht parallel zur Achse des Endoskops verläuft, an das distale Ende des Endoskops angesetzt zu werden. Dabei kann der Abschlussabschnitt gleichzeltig an den Schaftabschnitt angesetzt werden, mit dem er anschließend gefügt wird. Durch eine der vorbeschriebenen Ausbildungen des Mantels kann der Innenkörper mit Ausnahme der Fläche des Fensters vollständig umschlossen werden, nachdem das Fenster und der Innenkörper gefügt sind.

Bei einem Verfahren zum Herstellen eines Endoskops wird ein Fenster an ein distales Ende eines Innenkörpers gefügt, ein Mantel um den Instrumentenkörper angeordnet und eine Fuge zwischen dem Fenster und dem Mantel des Endoskops abgedichtet. Vor oder nach dem Abdichten der Fuge können ein Abschlussabschnitt, der das Fenster umgibt, und ein Schaftabschnitt des Mantels gefügt werden. Nach dem Fügen des Fensters an das distale Ende des Innenkörpers und vor dem Abdichten der Fuge kann der Abschlussabschnitt an einer Hinterschneidung an einem distalen Ende des Innenkörpers eingehängt und um eine Achse nahe der Hinterschneidung geschwenkt werden. Mit dem vorstehend beschriebenen Verfahren kann insbesondere eines der oben dargestellten Endoskope hergestellt werden.

Bei einem weiteren Verfahren zum Herstellen eines Endoskops werden ein Abschlussabschnitt, der ein Fenster umgibt, und ein Schaftabschnitt zu einem Mantel gefügt, der insbesondere von dem Fenster abgesehen die gesamte Oberfläche des distalen Endes des Endoskops bildendet. Vor dem Fügen kann der Abschlussabschnitt in einer geradlinigen oder nicht-geradlinigen, jedenfalls im letzten Abschnitt nicht zur Achse des Endoskops parallelen Bewegung an das distale Ende des Endoskops angesetzt werden. Diese Bewegung kann durch eine Rotationsbewegung überlagert oder ergänzt werden. Alternativ wird der Abschlussabschnitt beispielsweise zunächst an dem distalen Ende des Endoskops eingehängt und anschließend mit einer Schwenkbewegung in seine endgültige Lage relativ zu anderen Teilen des Endoskops gebracht.

### Kurzbeschreibung der Figuren

Nachfolgend werden Ausführungsformen anhand der beigefügten Figuren näher erläutert. Es zeigen:
- Figur 1: eine schematische Darstellung eines Endoskops;
- Figur 2: eine schematische axonometrische Darstellung eines Innenkörper eines Endoskops;
- Figur 3: eine schematische axonometrische Garstellung eines distalen Endes eines Endoskops;
- Figur 4: eine schematische axonometrische Darstellung eines distalen Endes eines Endoskops;
- Figur 5: ein schematisches Flussdiagramm eines Verfahrens zum Herstellen eines Endoskops.

### Beschreibung der Ausführungsformen

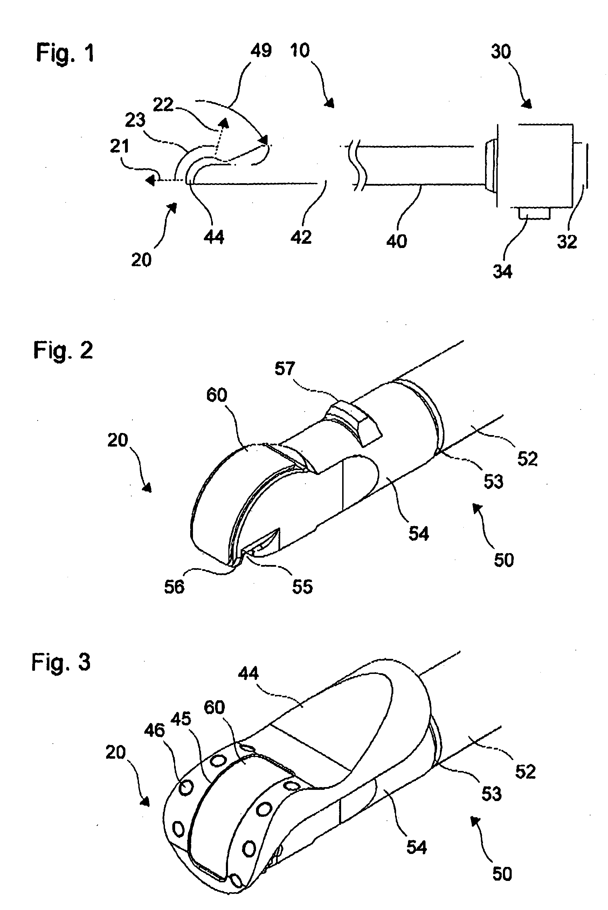

Figur 1 zeigt eine schematische Darstellung eines Endoskops 10 in einer seitlichen Ansicht. Eine Längsachse des Endoskops 10, entlang derer das Endoskop 10 eine große Ausdehnung aufweist, liegt parallel zur Zeichenebene. Die Länge des Endoskops 10 zwischen einem distalen Ende 20 und einem proximalen Ende 30 ist deutlich verkürzt dargestellt.

Das Endoskop 10 ist für variierbare Blickrichtungen zwischen einer ersten extremen Blickrichtung 21 und einer zweiten extremen Blickrichtung 22 ausgebildet, wobei die erste extreme Blickrichtung 21 näherungsweise parallel zur Achse des Endoskops 10 ist. Zur Variation der Blickrichtung weist das Endoskop 10 beispielsweise einen schwenkbaren Spiegel, ein schwenkbares Prisma oder eine schwenkbare Kamera, gegebenenfalls einen elektronischen Bildaufnehmer, im distalen Ende 20 auf. Der Winkelbereich 23 zwischen den extremen Blickrichtungen 21, 22 umfasst vorzugweise mehr als 90°, insbesondere 120°.

Das proximale Ende 30 des Endoskops 10 umfasst eine erste Kupplung 32 und eine zweite Kupplung 34 zur Kopplung des Endoskops 10 mit einer Kamera, einer Lichtquelle, einem Okular und/oder anderen in Figur 1 nicht dargestellten Einrichtungen.

Das Endoskop 10 umfasst einen Mantel 40, der vom proximalen Ende 30 abgesehen das Endoskop 10 wie nachfolgend näher beschirieben im Wesentlichen vollständig nach außen hin abschließt und seine äußere Oberfläche bildet. Der Mantel 40 umfasst einen im Wesentlichen rohrförmigen Schaftabschnitt 42 und einen Abschlussabschnitt 44 am distalen Ende 20.

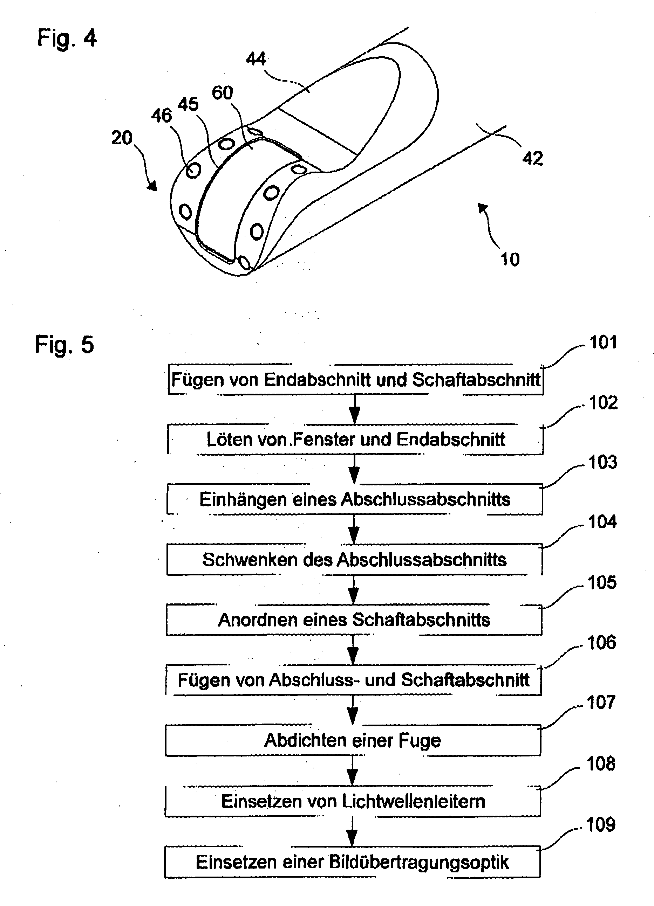

In Figur 1 ist ferner ein Pfeil 49 dargestellt, dessen Bedeutung erst unten im Zusammenhang mit den Figuren 3 und 5 erläutert wird.

Die Figuren 2 bis 4 zeigen schematische axonometrische Darstellungen des distalen Endes 20 des Endoskops 10 in verschiedenen Stadien seiner Herstellung.

Figur 2 zeigt eine schematische Darstellung eines Innenkörpers 50 mit einem Schaftabschnitt 52, der an einer Fügestelle 53 mit einem Endabschnitt 54 dauerhaft mechanisch verbunden ist, beispielsweise durch Schweißen oder Löten. Der Schaftabschnitt 52 weist beispielsweise die Form eines Rohres mit beispielsweise kreisringtörmigem Querschnitt auf. Der Endabschnitt 54 weist eine komplexere Form mit einem in Figur 2 nicht erkennbaren Hohlraum auf. Der Hohlraum im Endabschnitt 54 erstreckt sich vom distalen Ende 20 bis zum Schaftabschnitt 52, in dessen Lumen er übergeht. Der Endabschnitt 54 weist unmittelbar am distalen Ende 20 eine Nut 55 und einen Steg 56 auf Ferner weist der Endabschnitt 54 einen Nocken 57 an einer von der Nut 55 und dem Steg 56 beabstandeten Seite und nahe am Schaftabschnitt 52 auf.

Ferner weist der Endabschnitt 54 eine Öffnung zu dem genannten Hohlraum auf, die durch ein Fenster 60 verschlossen ist. Das Fenster 60 hat insbesondere die Form eines Ausschnitts eines kreiszylindrischen Hohlkörpers mit konstanter Wandstärke. Es kann aber auch aus einem Abschnitt eines in zwei Richtungen gekrümmten Hohlkörpers sein. In beiden Fällen kann die Wandstärke konstant oder auch variabel sein. Das Fenster 60 weist Saphir (insbesondere monokristallinen Saphir), einen anderen Korund oder ein anderes transparentes und insbesondere farbloses Material auf. Das Fenster 60 ist durch Hartlöten oder Weichlöten, insbesondere mittels eines Goldlots, an den Endabschnitt 54 gefügt und verschließt dessen Öffnung hermetisch.

Figur 3 zeigt das distale Ende 20 des Endoskops 10 nachdem der Abschlussabschnitt 44 des Mantels angesetzt wurde. Der Abschlussabschnitt 44 weist einen in Figur 3 nicht erkennbaren weil in dieser Perspektive verborgenen Steg auf, der in die in Figur 2 dargestellte Nut 55 hinter dem Steg 56 am Endabschnitt 54 eingreift. Zum Ansetzen des Abschlussabschnitts 44 wird dieser Steg am Abschlussabschnitt 44 vor der oder in die Nut 55 an- oder eingesetzt und dann der Abschlussabschnitt 44 mit einer durch den Pfeil 49 in Figur 1 dargestellten Schwenkbewegung in die in Figur 3 gezeigte Position gebracht. In der in Figur 3 dargestellten Lage des Abschlussabschnitts 44 liegt dieser an dem Nocken 57 an. Alternativ greift der Nocken 57 bei der in Figur 3 dargestellten Lage des Abschlussabschnitts 44 in eine entsprechende Ausnehmung im Abschlussabschnitt 44 ein.

Durch die Nut 55 und/oder den Steg 56 und den Nocken 57 am Endabschnitt 54 des Innenkörpers 50 einerseits und den oben genannten, in Figur 3 nicht sichtbaren Steg am Abschlussabschnitt 44 und gegebenenfalls die ebenfalls oben genannte Ausnehmung am Abschlussabschnitt 44 des Mantels 40 andererseits ist die relative Lage des Abschlussabschnitts 44 des Mantels 40 zum Endabschnitt 54 des Innenkörpers 50 definiert. In dieser Lage des Abschlussabschnitts 44 liegt das Fenster 60 in einer entsprechenden Öffnung des Abschlussabschnitts 44.

Zwischen dem Fenster 60 bzw. dessen äußerem Rand einerseits und dem Abschlussabschnitt 44 bzw. dem Rand der mit dem Fenster 60 korrespondierenden Öffnung andererseits wird eine Abdichtung angebracht, beispielsweise in Form eines O-Rings aus einem Elastomer und/oder in Form von Klebstoff, Silikon, Harz oder einem anderen Polymer, das in duktiler Form in den Zwischenraum zwischen dem Fenster 60 und dem Abschlussabschnitt 44 eingebracht wird und dort aushärtet.

An zwei gegenüberliegenden, parallel zu einer Ebene, die die Achse des Endoskops 10 enthält, angeordneten Seiten des Fensters 60 sind jeweils mehrere Lichtaustrittsöffnungen 46 angeordnet. Die Lichtaustrittsöffnungen 46 an einer Seite des Fensters 60 sind versetzt, gegebenenfalls auch symmetrisch, zum den Lichtaustrittsöffnungen 46 an der anderen Seite des Fensters 60 angeordnet. Die Lichtaustrittsöffnungen 46 sind beispielsweise mit Lichtwellenleitern gekoppelt, die entlang des Endoskops 10 vom proximalen Ende 30 zum distalen Ende 20 verlaufen. Die Öffnungswinkel und Ausrichtungen der einzelnen Lichtaustrittsöffnungen sind so gewählt, dass aus den Lichtaustrittsöffnungen 46 austretendes Licht einen mittels des Endoskops 10 betrachteten Hohlraum möglichst gleichmäßig ausleuchtet.

Figur 4 zeigt das distale Ende 20 des Endoskops 10 nachdem der Schaftabschnitt 42 des Mantels 40 in Richtung vom proximalen Ende 30 zum distalen Ende 20 über den Innenkörper 50 geschoben wurde. Bei der in Figur 4 dargestellten Lage des Schaftabschnitts 42 und des Abschlussabschnitts 44 liegen deren Ränder aneinander an und können gefügt, beispielsweise verschweißt, werden. Nach dem Fügen ist das Endoskop 20 - vom proximalen Ende 30 und der Fläche des Fensters 60 abgesehen - von dem Mantel 40 aus dem Schaftabschnitt 42 und dem Abschlussabschnitt 44 vollständig umschlossen. Anders ausgedrückt bildet der Mantel 40 - vorn proximalen Ende 30 und dem Fenster 60 abgesehen - die gesamte äußere Oberfläche des Endoskops 10. Bei Verwendung des Endoskops an einem menschlichen oder tierischen Körper kommt dieser deshalb nur mit dem Mantel 40 und dem Fenster 60, nicht jedoch mit dem Innenkörper 50 in Berührung.

An Stelle der oben anhand der Figuren 1 und 3 dargestellten Anbringung des Abschlussabschnitts 44 durch Einhängen oder Ansetzten in oder an der Nut 55 des Endabschnitts 54 und anschließendes Schwenken 49 kann der Abschlussabschnitt 44 auch beispielsweise in einer linearen Bewegung in der Zeichenebene der Figur 1 und in einem Winkel von ca. 60° zur Längsachse des Endoskops 10 an den Endabschnitt 54 des Innenkörpers 50 angesetzt werden.

Eine alternative Möglichkeit zur Anbringung des Abschlussabschnitts 44 ist eine zweiteilige Ausführung, bei der der Abschlussabschnitt 44 aus einem ersten Teil des Abschlussabschnitts 44 und einem zweiten Teil des Abschlussabschnitts 44 besteht und die beiden Teile jeweils von der entsprechenden Seite aufgeschoben oder rotatorisch aufgebracht werden und anschließend, z.B. durch Schweißen, gefügt werden.

Figur 5 zeigt ein schematisches Flussdiagramm eines Verfahrens zum Herstellen eines Endoskops. Obwohl mit dem Verfahren auch Endoskope hergestellt werden können, die sich von den oben anhand der Figuren 1 bis 4 dargestellten unterscheiden, werden nachfolgend beispielhaft Bezugszeichen aus den Figuren 1 bis 4 verwendet, um das Verständnis zu erleichtern.

Bei einem ersten Schritt 101 werden ein Endabschnitt 54 und ein Schaftabschnitt 52 eines Innenkörpers 50 gefügt, beispielsweise durch Schweißen oder Löten. Der Endabschnitt 54 weist einen Hohlraum auf, der am proximalen Ende des Endabschnitts 54 nach dem Fügen in ein Lumen des Schaftabschnitts 52 übergeht. Am gegenüberliegenden, distalen Ende des Endabschnitts 54 weist dieser eine Öffnung zu dem Hohlraum auf.

Bei einem zweiten Schritt 102 wird ein Fenster 60 in oder an die genannte Öffnung des Endabschnitts 54 gelötet oder auf andere Weise gefügt. Danach verschließt das Fenster 60 die Öffnung in dem Endabschnitt 54 hermetisch. Insbesondere ist die Fügeverbindung zwischen dem Fenster 60 und dem Endabschnitt 54 so, dass beim Autoklavieren auch bei den dabei üblichen Temperaturen von 140°C oder mehr und den dabei üblichen Drücken von mehreren Bar kein Wasserdampf in das Innere des Innenkörpers 50 eindringen kann. Um aus einer unterschiedlichen thermischen Ausdehnung des Fensters 60 und des Endabschnitts 54 resultierende mechanische Spannungen zu vermeiden oder zumindest zu minimieren, sind die Materialien des Fensters 60 und des Endabschnitts 54 so gewählt, dass ihre linearen Wärmeausdehnungskoeffizienten möglichst ähnlich sind.

Bei einem dritten Schritt 103 wird ein Abschlussabschnitt 44 in eine Hinterschneidung 55, 56 an dem Endabschnitt 54 eingehängt. Bei einem vierten Schritt 104 wird der Abschlussabschnitt 44 um eine Achse an oder nahe der Hinterschneidung geschwenkt und in die vorgesehene Lage gebracht. An Stelle des Einhängens 103 und Schwenkcns 104 kann der Abschlussabschnitt 44 beispielsweise durch eine lineare, zumindest am Ende nicht zur Achse des Endoskops 10 parallelen Bewegung an den Endabschnitt 54 des Innenkörpers 50 angesetzt werden.

Bei einem fünften Schritt 105 wird ein Schaftabschnitt 42 angrenzend an den Abschlussabschnitt 44 angeordnet. Dazu wird der Schaftabschnitt 42 insbesondere in axialer Richtung des Endoskops 10 über den Innenkörper 50 geschoben. Bei einem sechsten Schritt 106 werden Abschlussabschnitt 44 und Schaftabschnitt 42 gefügt, beispielsweise durch Schweißen oder Löten.

Bei einem siebten Schritt 107 wird eine Fuge zwischen einem Rand des Fensters 60 und einem Rand einer korrespondierenden Öffnung des Abschlussabschutts 44 abgedichtet, beispielsweise mittels eines O-Rings aus einem Elastomer und/oder durch Klebstoff, Silikon, Harz oder ein anderes Polymer. Der siebte Schritt 107 kann alternativ zusammen mit dem dritten Schritt oder vor dem fünften Schritt 105 oder vor dem sechsten Schritt 106 ausgeführt werden.

Bei einem achten Schritt 108 werden Lichtwellenleiter vom proximalen Ende 30 zu Lichtaustrittsöffnungen 46 am distalen Ende 20 des Endoskops 10 eingesetzt. Bei einem neunten Schritt 109 wird eine Bildübertragungsoptik, beispielsweise ein Stablinsensystem nach Hopkins, in den Innenkörper 50 des Endoskops 10 eingesetzt. Der achte Schritt 108 und der neunte Schritt 109 können in umgekehrter Reihenfolge ausgeführt werden. Ferner kann sowohl der achte Schritt 108 als auch der neunte Schritt 109 zu einem früheren Zeitpunkt ausgeführt werden, insbesondere vor dem dritten Schritt 103.

Der dritte Schritt 103, der vierte Schritt 104 oder die beschriebene Alternative zu diesen beiden Schritten sowie der fünfte Schritt 105 und der sechste Schritt 106 können als eigenständiges Verfahren ausgeführt werden, unabhängig vom ersten Schritt 101, dem zweiten Schritt 102, dem siebten Schritt 107, dem achten Schritt 108 und dem neunten Schritt 109.

### Bezugszeichen

- 10: Endoskop
- 20: distales Ende des Endoskops 10
- 21: erste extreme Blickrichtung
- 22: zweite extreme Blickrichtung
- 23: Winkelbereich der Blickrichtungen
- 30: proximales Ende des Endoskops 10
- 32: erste Kupplung
- 34: zweite Kupplung
- 40: Mantel
- 42: Schaftabschnitt des Mantels 40
- 44: Abschlussabschnitt des Mantels 40
- 45: Abdichtung zwischen Abschlussabschnitt 44 und Fenster 60
- 46: Lichtaustrittsöffnung
- 49: Schwenkbewegung
- 50: Innenkörper
- 52: Schaftabschnitt des Innenkörper 50
- 53: Fügestelle des Innenkörpers 50
- 54: Endabschnitt des Innenkörpers 50
- 55: Nut am Endabschnitt 54
- 56: Steg am Endabschnitt 54
- 57: Nocken am Endabschnitt 54
- 60: Fenster

- 101: erster Schritt
- 102: zweiter Schritt
- 103: dritter Schnitt
- 104: vierter Schritt
- 105: fünfter Schritt
- 106: sechster Schritt
- 107: siebter Schritt
- 108: achter Schritt
- 109: neunter Schritt

## Patentansprüche

1. **Endoskop** (10) mit:
einem **Fenster** (60) an einem distalen Ende (20) des Endoskops (10);
einem **Innenkörper** (50) mit einem **ersten Material** am distalen Ende (20), der keine äußeren Oberflächen des Endoskops bildet;
einem **Mantel** (40) mit einem **zweiten Material** am distalen Ende (20),
wobei das Fenster (60) und der Innenkörper (50) gefügt sind, und wobei eine **Klebenaht** oder eine Dichtung zwischen dem Rand des Fensters (60) und dem Mantel (40) vorgesehen ist, **gekennzeichnet dadurch, dass**
eine Differenz der **thermischen Ausdehnungskoeffizienten** des ersten Materials und eines Materials des Fensters (60) kleiner ist als eine Differenz der thermischen Ausdehnungskoeffizienten des zweiten Materials und des Materials des Fensters (60).

2. Endoskop (10) nach einem der vorangehenden Ansprüche, bei dem das **Fenster** (60) zumindest in einer Richtung **gekrümmt** ist.

3. Endoskop (10) nach einem der vorangehenden Ansprüche, bei dem das Fenster (60) eine Öffnung an dem Innenkörper (50) hermetisch verschließt.

4. Endoskop (10) nach einem der vorangehenden Ansprüche, bei dem das Fenster (60) und der Innenkörper (50) **verlötet** sind.

5. Endoskop (10) nach einem der vorangehenden Ansprüche, bei dem das erste Material eine **Einschmeilzlegierung** ist.

6. Endoskop (10) nach einem der vorangehenden Ansprüche, bei dem das Fenster (60) Saphir oder einen anderen Korund aufweist.

7. Endoskop (10) nach einem der vorangehenden Ansprüche, bei dem
der Mantel (40) einen **Abschlussabschnitt** (44) am distalen Ende (20) und einen **Schaftabschnitt** (42) umfasst,
der Abschlussabschnitt (44) und der Schaftabschnitt (42) gefügt sind.

8. Endoskop (10) nach einem der vorangehenden Ansprüche, ferner mit jeweils mehreren **Lichtaustrittsöffnungen** (46) an zwei gegenüberliegenden Seiten des Fensters (60), wobei die Lichtaustrittsöffnungen (46) an einer Seite des Fensters (60) **versetzt** zu den Lichtaustrittsöffnungen (46) an der anderen Seite des Fensters (60) angeordnet sind.

9. Verfahren zum Herstellen eines Endoskops (10) nach einem der vorangehende Ansprüche, mit folgenden Schritten:
**Fügen** (102) eines Fensters (60) an ein distales Ende (20) eines Innenkörpers (50);
Umschließen des Innenkörpers (50) mit einem Mantel (40);
**Abdichten** (107) einer Fuge zwischen dem Fenster (60) und dem Mantel (40) des Endoskops (10);
**Fügen** (106) eines Abschlussabschnitts (44) und eines Schaftabschnitts (42) des **Mantels** (40).

10. Verfahren nach dem vorangehenden Verfahrensanspruch, ferner mit folgenden Schritten, die nach dem Löten (102) und vor dem Abdichten (107) ausgeführt werden:
**Einhängen** (104) des Abschlussabschnitts (44) an einer Hinterschneidung (55, 56) an einem distalen Ende (20) des Innenkörpers (50);
**Schwenken** (105) des Abschlussabschnitts (44) um eine Achse nahe der Hinterschneidung (55, 56).

11. Verfahren nach dem vorangehenden Verfahrensanspruch.
ferner mit folgendem Schritt:
Einhängen (103) des Abschlussabschnitts (44) nach dem Einsetzen der Lichtleiter in Lichtaustrittsöffnungen des Abschlussabschnitts (44).

## Claims

1. Endoscope (10) with:
a window (60) at a distal end (20) of the endoscope (10);
an inner body (50) with a first material at the distal end (20), which inner body (50) does not form any outer surfaces of the endoscope;
a barrel (40) with a second material at the distal end (20),
the window (60) and the inner body (50) being joined, and an adhesive seam or a seal being provided between the edge of the window (60) and the barrel (40),
**characterized in that** a difference in the coefficients of thermal expansion of the first material and of a material of the window (60) is smaller than a difference in the coefficients of thermal expansion of the second material and of the material of the window (60).

2. Endoscope (10) according to one of the preceding claims, in which the window (60) is curved in at least one direction.

3. Endoscope (10) according to one of the preceding claims, in which the window (60) hermetically closes an opening on the inner body (50).

4. Endoscope (10) according to one of the preceding claims, in which the window (60) and the inner body (50) are soldered together.

5. Endoscope (10) according to one of the preceding claims, in which the first material is a sealing alloy.

6. Endoscope (10) according to one of the preceding claims, in which the window (60) is of sapphire or another corundum.

7. Endoscope (10) according to one of the preceding claims, in which
the barrel (40) comprises a terminal portion (44) at the distal end (20) and a shank portion (42),
the terminal portion (44) and the shank portion (42) being joined.

8. Endoscope (10) according to one of the preceding claims, also with several light outlet openings (46) on each of two opposite sides of the window (60), wherein the light outlet openings (46) on one side of the window (60) are arranged offset in relation to the light outlet openings (46) on the other side of the window (60).

9. Method for producing an endoscope (10) according to one of the preceding claims, with the following steps:
joining (102) a window (60) to a distal end (20) of an inner body (50);
enclosing the inner body (50) with a barrel (40);
sealing (107) a joint between the window (60) and the barrel (40) of the endoscope (10);
joining (106) a terminal portion (44) and a shank portion (42) of the barrel (40).

10. Method according to the preceding method claim, also with the following steps, which are carried out after the soldering (102) and before the sealing (107):
hooking (104) the terminal portion (44) on an undercut (55, 56) at a distal end (20) of the inner body (50);
pivoting (105) the terminal portion (44) about an axis near the undercut (55, 56).

11. Method according to the preceding method claim, also with the following step:
hooking (103) the terminal portion (44) after the insertion of light guides into light outlet openings of the terminal portion (44).

## Revendications

1. Endoscope (10) avec:
- une fenêtre (60) à une extrémité distale (20) de l'endoscope (10);
- un corps intérieur (50) avec un premier matériau à l'extrémité distale (20), qui ne forme pas de surfaces extérieures de l'endoscope;
- une enveloppe (40) avec un deuxième matériau à l'extrémité distale (20),
dans lequel la fenêtre (60) et le corps intérieur (50) sont joints, et dans lequel il est prévu un joint collé ou un joint d'étanchéité entre le bord de la fenêtre (60) et l'enveloppe (40),
**caractérisé en ce qu'**une différence entre les coefficients de dilatation thermique du premier matériau et d'un matériau de la fenêtre (60) est plus petite qu'une différence entre les coefficients de dilatation thermique du deuxième matériau et du matériau de la fenêtre (60).

2. Endoscope (10) selon l'une quelconque des revendications précédentes, dans lequel la fenêtre (60) est courbée au moins dans une direction.

3. Endoscope (10) selon l'une quelconque des revendications précédentes, dans lequel la fenêtre (60) ferme hermétiquement une ouverture sur le corps intérieur (50).

4. Endoscope (10) selon l'une quelconque des revendications précédentes, dans lequel la fenêtre (60) et le corps intérieur (50) sont brasés.

5. Endoscope (10) selon l'une quelconque des revendications précédentes, dans lequel le premier matériau est un alliage fusible.

6. Endoscope (10) selon l'une quelconque des revendications précédentes, dans lequel la fenêtre (60) présente un saphir ou un autre corindon.

7. Endoscope (10) selon l'une quelconque des revendications précédentes, dans lequel l'enveloppe (40) comprend une partie de fermeture (44) à l'extrémité distale (20) et une partie de tige (42), la partie de fermeture (44) et la partie de tige (42) sont jointes.

8. Endoscope (10) selon l'une quelconque des revendications précédentes, avec en outre chaque fois plusieurs ouvertures de sortie de lumière (46) sur deux côtés opposés de la fenêtre (60), dans lequel les ouvertures de sortie de lumière (46) sur un côté de la fenêtre (60) sont disposées en décalage par rapport aux ouvertures de sortie de lumière (46) sur l'autre côté de la fenêtre (60).

9. Procédé de fabrication d'un endoscope (10) selon l'une quelconque des revendications précédentes, comprenant les étapes suivantes:
- joindre (102) une fenêtre (60) à une extrémité distale (20) d'un corps intérieur (50);
- enfermer le corps intérieur (50) dans une enveloppe (40) ;
- colmater (107) un joint entre la fenêtre (60) et l'enveloppe (40) de l'endoscope (10);
- joindre (106) une partie de fermeture (44) et une partie de tige (42) de l'enveloppe (40).

10. Procédé selon la revendication de procédé précédente, comprenant en outre les étapes suivantes, qui sont exécutées après le brasage (102) et avant le colmatage (107):
- accrocher (104) la partie de fermeture (44) à une contre-dépouille (55, 56) à une extrémité distale (20) du corps intérieur (50);
- faire pivoter (105) la partie de fermeture (44) autour d'un axe à proximité de la contre-dépouille (55, 56).

11. Procédé selon la revendication de procédé précédente, comprenant en outre l'étape suivante:
- accrocher (103) la partie de fermeture (44) après l'insertion des guides d'onde dans des ouvertures de sortie de lumière de la partie de fermeture (44).
